(19) Europäisches Patentamt
European Patent Office
Office européen des brevets

(11) EP 3 894 399 B1

# (12) EUROPEAN PATENT SPECIFICATION

(45) Date of publication and mention of the grant of the patent:
**07.09.2022 Bulletin 2022/36**

(21) Application number: **19817307.2**

(22) Date of filing: **12.12.2019**

(51) International Patent Classification (IPC):
***C07D 309/04*** (2006.01) ***A61K 8/49*** (2006.01)

(52) Cooperative Patent Classification (CPC):
**C07D 309/04; A23L 27/72; A61K 8/498; A61Q 13/00; A61Q 19/00; C11B 9/00;**
A61K 2800/10

(86) International application number:
**PCT/EP2019/084773**

(87) International publication number:
**WO 2020/120643 (18.06.2020 Gazette 2020/25)**

(54) **(2S,4R)-4-METHYL-2-(2-METHYLBUT-1-EN-1-YL)TETRAHYDRO-2H-PYRAN AS PERFUME COMPONENT**

(2S,4R)-4-METHYL-2-(2-METHYLBUT-1-EN-1-YL)TETRAHYDRO-2H-PYRANEN ALS PARFÜMSTOFF

(2S,4R)-4-METHYL-2-(2-METHYLBUT-1-EN-1-YL)TETRAHYDRO-2H-PYRANEN TANT QUE COMPOSANT DE PARFUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.12.2018 EP 18212350**

(43) Date of publication of application:
**20.10.2021 Bulletin 2021/42**

(73) Proprietor: **Givaudan SA**
**1214 Vernier (CH)**

(72) Inventors:
- **LINIGER, Marc**
  **8424 Embrach (CH)**
- **NATSCH, Andreas**
  **8707 Uetikon (CH)**

(74) Representative: **Global Patents**
**Givaudan SA**
**Grafenaustrasse 7**
**6300 Zug (CH)**

(56) References cited:
**EP-A1- 0 082 401** **EP-A1- 2 112 144**
**EP-A2- 0 770 670** **EP-B1- 0 082 401**
**EP-B1- 0 770 670**

- **"Tetrahydro-4-methyl-2-(2-methylpropen-1-Y L)pyran ED - Verhagen Hans; Leino Olli", FOOD AND CHEMICAL TOXICOLOGY, PERGAMON, GB, vol. 30, 1 January 1992 (1992-01-01), pages 121-122, XP023810987, ISSN: 0278-6915 [retrieved on 1992-01-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 894 399 B1

## Description

### TECHNICAL FIELD

The present invention is concerned with fragrance ingredients and with fragrance preparations, for imparting desirable odor notes to consumer products, in particular it is concerned with fragrance ingredients and fragrance compositions that can be used as replacements in whole or in part for the recently disputed compound Rose Oxide (4-methyl-2-(2-methylprop-1-en-1-yl)tetrahydro-2H-pyran).

### BACKGROUND

Rose Oxide is a key fragrance ingredient possessing a floral, rosy, green natural odor profile with some metallic side notes and is widely used in perfumery. This compound has found wide use in fine perfumery as well as in personal and household care products. It is an essential ingredient to create floral and rosy accords. However, its use is controversial in view of recent findings that it exhibits toxic effects on the reproductive organs of male rats. Thus toxicological data registered on the REACH platform in the ECHA registration dossier of Rose Oxide indicate that in male rats treated with 300 or 1000 mg/kg body weight (bw) per day of Rose Oxide, sperm function and morphology in the cauda epididymis were adversely affected and sperm numbers were significantly reduced. A significant weight increase (relative +26%, absolute +33%) of the epididymis of males of the test group with 1000 mg/kg body weight per day was observed.

Histopathological assessment of the left epididymis revealed the presence of immature ducts in the distal corpus and/or cauda epididymis in all males of test groups with 300 and 1000 mg/kg body weight per day. Immature ducts increased in severity, ranging from minimal to slight in test group with 300 mg/kg body weight per day to slight to severe in test group with 1000 mg/kg body weight per day. With increasing severity the immature ducts were accompanied by increasing interstitial edema, which correlated with the significant weight increase found in test group at 1000 mg/kg body weight per day. These data are summarized on https://echa.europa.eu/de/registration-dossier/- /registered-dossier/19099/7/9/1.

The chemical information website states that "according to the classification provided by companies to ECHA in REACH registrations this substance [Rose Oxide] is suspected of damaging fertility or the unborn child" (https://echa.europa.eu/de/substance-information/-/substanceinfo/100.036.763). Thus the compound as such is classified as CMR2 and fragrance compositions containing 3wt% or more Rose Oxide have to be labeled under the EU regulations.

### SUMMARY OF THE INVENTION

In accordance with the first aspect of the present invention there is provided (2,4R)-4-methyl-2((Z)-2-methylbute-1-en-1-yl)tetrahydro-2 H-pyran.

In accordance with the second aspect of the present invention there is provided a fragrance composition comprising (2,4R)-4-methyl-2((Z)-2-methylbute-1-en-1-yl)tetrahydro-2H-pyran.

In accordance with the third aspect of the present invention there is provided a fragranced article comprising (2,4R)-4-methyl-2((Z)-2-methylbute-1-en-1-yl)tetrahydro-2H-pyran, and a consumer product base.

In accordance with the fourth aspect of the present invention there is provided a method of manufacturing a fragranced article, comprising the incorporation of (2S,4R)-4-methyl-2-((Z)-2-methylbut-1-en-1-yl)tetrahydro-2H-pyran as a fragrance ingredient, either by directly admixing to the consumer product base or by admixing a fragrance composition comprising (2S,4R)-4-methyl-2-((Z)-2-methylbut-1-en-1-yl)tetrahydro-2H-pyran which may then be mixed with a consumer product base, using conventional techniques and methods.

In accordance with the fifth aspect of the present invention there is provided a method for improving, enhancing or modifying a consumer product base by means of the addition thereto of an olfactorily acceptable amount of (2S,4R)-4-methyl-2-((Z)-2-methylbut-1-en-1-yl)tetrahydro-2H-pyran.

In accordance with the sixth aspect of the present invention there is provided a method of replacing 4-methyl-2-(2-methylprop-1-en-1-yl)tetrahydro-2H-pyran in a fragrance composition, said method comprising employing in the fragrance composition (2S,4R)-4-methyl-2-((Z)-2-methylbut-1-en-1-yl)tetrahydro-2H-pyran, and at least one additional fragrance ingredient, so that the level of 4-methyl-2-(2-methylprop-1-en-1-yl)tetrahydro-2H-pyran, if present in said fragrance composition, is less than 0.1%, e.g. less than 0.01% or free of 4-methyl-2-(2-methylprop-1-en-1-yl)tetrahydro-2H-pyran.

### DETAILED DESCRIPTION

There is a need to provide ingredients that can be used in fragrance compositions as a replacement for Rose Oxide or which may be used in fragrance compositions to reduce substantially the amount of Rose Oxide in such

compositions.

For economic reasons the Rose Oxide quality used in the flavour and fragrance industry is the racemic mixture of 4-methyl-2-(2-methylprop-1-en-1-yl)tetrahydro-2H-pyran and thus constitutes the benchmark.

Fragrance ingredients possessing a floral, rosy, green odor profile include 4-methyl-2-phenyltetrahydro-2H-pyran (Doremox®), 2-isobutyl-4-methyltetrahydro-2H-pyran (Dihydroroseoxide), 4-methyl-1-oxaspiro[5.5]undec-4-ene (Oxaspirane), 2-butyl-4,6-dimethyl-3,6-dihydro-2H-pyran (Gyrane), and alpha-cyclohexylidene benzeneacetonitrile (Peonile®).

However, the use of each of these ingredients is not without its problems. For example, Doremox® does possess a similar odor profile but is too long-lasting compared to Rose Oxide. Dihydroroseoxide possesses the green facet, but is too fresh terpenic-terpineol, and less rosy-fruity.

To be suitable as a complete replacement for Rose Oxide not only the regulatory concerns associated with this compound have to be overcome, but also the odor profile and the evaporation profile of a compound or mixture of compounds should be very similar to Rose Oxide. Only then the replacement of Rose Oxide in fragrance composition will be possible without noticeable change of the overall odor characteristics. In case that not essentially all odor characteristics (odor profile and evaporation profile) are very similar to Rose Oxide a replacement is only possible in combination with reformulation of a fragrance composition. Rose Oxide is a highly volatile top note, which does not last long. In general it evaporates on a smelling strip within 30 minutes.

Applicant surprisingly found that *rel*-(2*S*,4*R*)-4-methyl-2-(2-methylbut-1-en-1-yl)tetrahydro-2H-pyran does not supress sperm formation in rats and at the same time it possesses substantially similar odor characteristics (odor profile and evaporation profile) as Rose Oxide. With 4-methyl-2-(2-methylbut-1-en-1-yl)tetrahydro-2H-pyran a compound was found possessing very similar odor characteristics to Rose Oxide without the drawback of toxicity concerns.

4-Methyl-2-(2-methylbut-1-en-1-yl)tetrahydro-2H-pyran has only be described once in the literature 40 years ago by W Rohan and W. Bruhn (Dragoco Report (German Edition) (1978), 25(11-12), 248-253). According to the report the isomeric mixture of 4-methyl-2-(2-methylbut-1-en-1-yl)tetrahydro-2H-pyran was described having "a somewhat burned smell, in the after-smell something celery-like, and an odor faintly reminiscent of (-)Rose Oxide ". Both, the burned smell as well as the celery-like after-smell indicate strongly, that 4-methyl-2-(2-methylbut-1-en-1-yl)tetrahydro-2H-pyran is not suitable as a replacement for Rose Oxide.

4-Methyl-2-(2-methylbut-1-en-1-yl)tetrahydro-2H-pyran comprises 2 stereocenters in the ring and attached to the C-2 position of the ring a branched asymmetric alkene substituent (i.e. E/Z isomers) thus resulting in 8 stereoisomers as shown below.

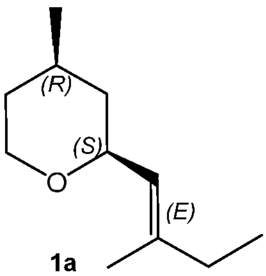
**1a**

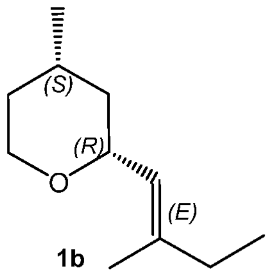
**1b**

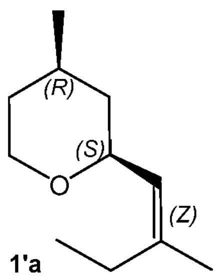
**1'a**

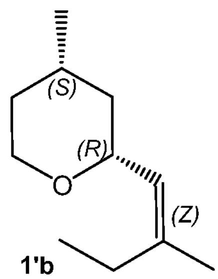
**1'b**

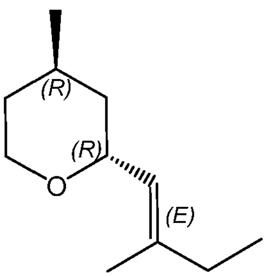
**2a**

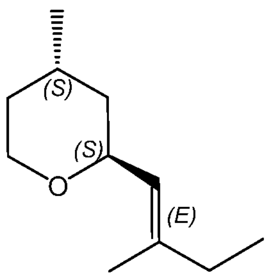
**2b**

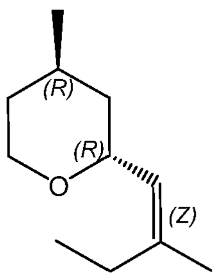
**2'a**

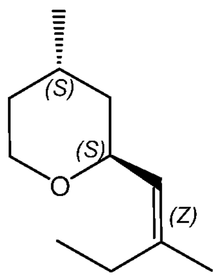
**2'b**

It was found that the stereoisomer (1'a) constitutes the main odor vector, whereas all other stereoisomers are either odourless or possess only a very weak odor.

Thus, there is provided in one aspect of the present invention a compound of (2S,4R)-4-methyl-2-((Z)-2-methylbut-1-en-1-yl)tetrahydro-2H-pyran (1'a) having a strong rosy, rose oxide, metallic odor.

However, since the resolution of the enantiomers and E/Z stereoisomers increases the complexity of the production, it is preferred to use an E/Z mixture of *rel*-(2*S*,4*R*)-4-methyl-2-(2-methylbut-1-en-1-yl)tetrahydro-2H-pyran (i.e. a mixture of 1a, 1b, 1'a and 1'b) simply for economic reasons.

In one embodiment the E/Z mixture of compounds of formula (1) and compounds of formula (1') is enriched in (1') ((1') is a mixture of compounds (1'a) and (1'b)), preferably in the ratio from 51 : 49 or greater (e.g. in a ratio of 55 : 45, 60 : 40, 70 : 30, 80 : 20 of compounds of formula (1') : compounds of formula (1)).

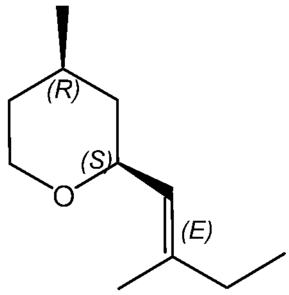

(1)
*rel*-(2*S*,4*R*)-(*E*)

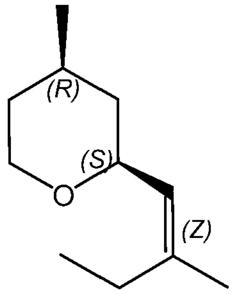

(1')
*rel*-(2*S*,4*R*)-(*Z*)

In another embodiment the cis/trans mixture of compounds of formula (1') and compounds of formula (2') is enriched in (1') ((1') a mixture of compounds (1'a) and (1'b)), preferably in the ratio from 51: 49 95 : 5, or greater (e.g. in a ratio of 60:40, 70:30, 80:20, 90:10, 95:5 of compounds of formula (1') : compounds of formula (2')).

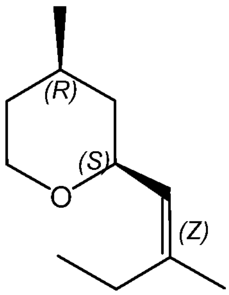

(1')
*rel*-(2*S*,4*R*)-(*Z*)

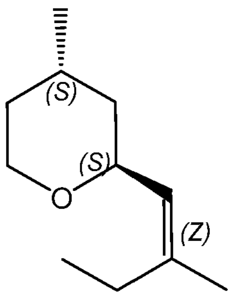

(2')
*rel*-(2*R*,4*R*)-(*Z*)

(2*S*,4*R*)-4-Methyl-2-((Z)-2-methylbut-1-en-1-yl)tetrahydro-2H-pyran may be used alone, as isomeric mixture thereof, or in combination with known odorant molecules selected from the extensive range of natural products, and synthetic molecules currently available, such as essential oils, alcohols, aldehydes and ketones, ethers and acetals, esters and lactones, macrocycles and heterocycles, and/or in admixture with one or more ingredients or excipients conventionally used in conjunction with odorants in fragrance compositions, for example, carrier materials, and other auxiliary agents commonly used in the art.

As used herein, "carrier material" means a material which is practically neutral from a odorant point of view, i.e. a material that does not significantly alter the organoleptic properties of odorants.

The term "auxiliary agent" refers to ingredients that might be employed in a fragrance composition for reasons not specifically related to the olfactive performance of said composition. For example, an auxiliary agent may be an ingredient that acts as an aid to processing a fragrance ingredient or ingredients, or a composition containing said ingredient(s), or it may improve handling or storage of a fragrance ingredient or composition containing same. It might also be an ingredient that provides additional benefits such as imparting color or texture. It might also be an ingredient that imparts light resistance or chemical stability to one or more ingredients contained in a fragrance composition. A detailed description of the nature and type of adjuvants commonly used in fragrance compositions containing same cannot be exhaustive, but it has to be mentioned that said ingredients are well known to a person skilled in the art.

As used herein, 'fragrance composition' means any composition comprising (2S,4R)-4-methyl-2-((Z)-2-methylbut-1-en-1-yl)tetrahydro-2H-pyran and a base material, e.g. a diluent conventionally used in conjunction with odorants, such as diethyl phthalate (DEP), dipropylene glycol (DPG), isopropyl myristate (IPM), pentane-1,2-diol, triethyl citrate (TEC) and alcohol (e.g. ethanol). Optionally, the composition may comprise an anti-oxidant adjuvant. Said anti-oxidant

may be selected from Tinogard® TT (BASF), Tinogard® Q (BASF), Tocopherol (including its isomers, CAS 59-02-9; 364-49-8; 18920-62-2; 121854-78-2), 2,6-bis(1,1-dimethylethyl)-4-methylphenol (BHT, CAS 128-37-0) and related phenols, hydroquinones (CAS 121-31-9).

The following list comprises examples of known odorant molecules, which may be combined with (2S,4R)-4-methyl-2-((Z)-2-methylbut-1-en-1-yl)tetrahydro-2H-pyran

- essential oils and extracts, e.g. castoreum, costus root oil, oak moss absolute, geranium oil, tree moss absolute, basil oil, fruit oils, such as bergamot oil and mandarine oil, myrtle oil, palmarose oil, patchouli oil, petitgrain oil, jasmine oil, rose oil, sandalwood oil, wormwood oil, lavender oil and/ or ylang-ylang oil;

- alcohols, e.g. cinnamic alcohol ((*E*)-3-phenylprop-2-en-1-ol); cis-3-hexenol ((*Z*)-hex-3-en-1-ol); citronellol (3,7-dimethyloct-6-en-1-ol); dihydro myrcenol (2,6-dimethyloct-7-en-2-ol); Ebanol™ ((*E*)-3-methyl-5-(2,2,3-trimethylcyclopent-3-en-1-yl)pent-4-en-2-ol); eugenol (4-allyl-2-methoxyphenol); ethyl linalool ((*E*)-3,7-dimethylnona-1,6-dien-3-ol); farnesol ((2E,6Z)-3,7,11-trimethyldodeca-2,6,10-trien-1-ol); geraniol ((*E*)-3,7-dimethylocta-2,6-dien-1-ol); Super Muguet™ ((*E*)-6-ethyl-3-methyloct-6-en-1-ol); linalool (3,7-dimethylocta-1,6-dien-3-ol); menthol (2-isopropyl-5-methylcyclohexanol); Nerol (3,7-dimethyl-2,6-octadien-1-ol); phenyl ethyl alcohol (2-phenylethanol); RhodinolTM (3,7-dimethyloct-6-en-1-ol); Sandalore™ (3-methyl-5-(2,2,3-trimethylcyclopent-3-en-1-yl)pentan-2-ol); terpineol (2-(4-methylcyclohex-3-en-1-yl)propan-2-ol); or Timberol™ (1-(2,2,6-trimethylcyclohexyl)hexan-3-ol); 2,4,7-trimethylocta-2,6-dien-1-ol, and/or [1-methyl-2(5-methylhex-4-en-2-yl)cyclopropyl]-methanol;

- aldehydes and ketones, e.g. anisaldehyde (4-methoxybenzaldehyde); alpha amyl cinnamic aldehyde (2-benzylideneheptanal); Georgywood™ (1-(1,2,8,8-tetramethyl-1,2,3,4,5,6,7,8-octahydronaphthalen-2-yl)ethanone); Hydroxycitronellal (7-hydroxy-3,7-dimethyloctanal); Iso E Super® (1-(2,3,8,8-tetramethyl-1,2,3,4,5,6,7,8-octahydronaphthalen-2-yl)ethanone); Isoraldeine® ((*E*)-3-methyl-4-(2,6,6-trimethylcyclohex-2-en-1-yl)but-3-en-2-one); Hedione® (methyl 3-oxo-2-pentylcyclopentaneacetate); 3-(4-isobutyl-2-methylphenyl)propanal; maltol; methyl cedryl ketone; methylionone; verbenone; and/or vanillin;

- ether and acetals, e.g. Ambrox® (3a,6,6,9a-tetramethyl-2,4,5,5a,7,8,9,9b-octahydro-1*H*-benzo[e][1]benzofuran); geranyl methyl ether ((2*E*)-1-methoxy-3,7-dimethylocta-2,6-diene); and/ or Spirambrene® (2',2',3,7,7-pentamethylspiro[bicyclo[4.1.0]heptane-2,5'-[1,3]dioxane]) ;

- esters and lactones, e.g. benzyl acetate; cedryl acetate ((1*S*,6*R*,8a*R*)-1,4,4,6-tetramethyloctahydro-1*H*-5,8a-methanoazulen-6-yl acetate); γ-decalactone (6-pentyltetrahydro-2*H*-pyran-2-one); Helvetolide® (2-(1-(3,3-dimethylcyclohexyl)ethoxy)-2-methylpropyl propionate); γ-undecalactone (5-heptyloxolan-2-one); and / or vetiveryl acetate ((4,8-dimethyl-2-propan-2-ylidene-3,3a,4,5,6,8a-hexahydro-1*H*-azulen-6-yl) acetate);

- macrocycles, e.g. Ambrettolide ((*Z*)-oxacycloheptadec-10-en-2-one); ethylene brassylate (1,4-dioxacycloheptadecane-5,17-dione); and / or Exaltolide® (16-oxacyclohexadecan-1-one); and

- heterocycles, e.g. isobutylquinoline (2-isobutylquinoline).

Thus there is provided in a further aspect of the invention a fragrance composition comprising (2S,4R)-4-methyl-2-((Z)-2-methylbut-1-en-1-yl)tetrahydro-2H-pyran.

Preferably, fragrance compositions comprising (2S,4R)-4-methyl-2-((Z)-2-methylbut-1-en-1-yl)tetrahydro-2H-pyran contain less than 0.1wt% (e.g. less than 0.01wt% or free of) of 4-methyl-2-(2-methylprop-1-en-1-yl)tetrahydro-2H-pyran.

In one particular embodiment fragrance compositions comprising (2S,4R)-4-methyl-2-((Z)-2-methylbut-1-en-1-yl)tetrahydro-2H-pyran are free of (or essentially free of) 4-methyl-2-(2-methylprop-1-en-1-yl)tetrahydro-2 H-pyran.

In a further embodiment the fragrance composition comprises an E/Z mixture of rel-(2*S*,4*R*)-4-methyl-2-(2-methylbut-1-en-1-yl)tetrahydro-2H-pyran in the ratio from 51 : 49 to 80 : 20, or greater (e.g. in a ratio of 55 : 45, 60 : 40, 70 : 30, 80 : 20 of compounds of formula (1') : compounds of formula (1)).

In certain embodiments the fragrance composition comprises an E/Z mixture of rel-(2*S*,4*R*)-4-methyl-2-(2-methylbut-1-en-1-yl)tetrahydro-2H-pyran in the ratio from 51: 49 to 80 : 20, or greater (e.g. in a ratio of 55 : 45, 60 : 40, 70 : 30, 80 : 20 of compounds of formula (1): compounds of formula (1')).

In certain embodiments the fragrance composition comprises a cis/trans mixture of compounds of formula (1') and compounds of formula (2') in the ratio from 51 : 49 to 95 : 5, or greater (e.g. in a ratio of 60:40, 70:30, 80:20, 90:10, 95:5 of compounds of formula (1') : compounds of formula (2')).

(2*S*,4*R*)-4-Methyl-2-((Z)-2-methylbut-1-en-1-yl)tetrahydro-2H-pyran may be used in a broad range of fragranced articles, e.g. in any field of fine and functional perfumery, such as perfumes, air care products, household products, laundry products, body care products and cosmetics. The compound can be employed in widely varying amounts, depending upon the specific article and on the nature and quantity of other odorant ingredients. The proportion is typically from 0.00001 to 3 weight per cent of the article. In one embodiment, the compound may be employed in a fabric softener in an amount from 0.0001 to 0.3 weight per cent (e.g. 0.001 to 0.1 including 0.05 weight %). In another embodiment, the compound may be used in fine perfumery in amounts from 0.01 to 30 weight per cent (e.g. up to about 10 or up to 20 weight per cent), more preferably between 0.01 and 5 weight per cent (e.g. 0.01 to 0.1 weight per cent). However, these values are given only by way of example, since the experienced perfumer may also achieve effects or may create novel accords with lower or higher concentrations.

In one embodiment there is provided a fragranced article comprising an acceptable amount of (2S,4R)-4-methyl-2-((Z)-2-methylbut-1-en-1-yl)tetrahydro-2H-pyran. For example, the fragrance article may comprise 0.0000001weight % to 90 weight % (including 0.000001 weight %; 0.00001weight %, 0.0001 weight %, 0.001 weight %, 0.01 weight %, 0.05 weight %, 0.1 weight %, 0.5 weight %, 1 weight %, 5 weight %, 8 weight %, 10 weight %, 15 weight %, 20 weight %, 25 weight %, 30 weight %, 50 weight %, 60 weight %, 65 weight %) based on the total amount of the article.

(2S,4R)-4-methyl-2-((Z)-2-methylbut-1-en-1-yl)tetrahydro-2H-pyran may be employed in a consumer product base simply by directly mixing the compound, or a fragrance composition comprising (2S,4R)-4-methyl-2-((Z)-2-methylbut-1-en-1-yl)tetrahydro-2H-pyran, or an isomeric mixture thereof, with the consumer product base, or it may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, and then mixed with the consumer product base.

Thus, the invention additionally provides a method of manufacturing a fragranced article, comprising the incorporation of (2S,4R)-4-methyl-2-((Z)-2-methylbut-1-en-1-yl)tetrahydro-2H-pyran as a fragrance ingredient, either by directly admixing to the consumer product base or by admixing a fragrance composition comprising (2S,4R)-4-methyl-2-((Z)-2-methylbut-1-en-1-yl)tetrahydro-2H-pyran, which may then be mixed with a consumer product base, using conventional techniques and methods. Through the addition of an olfactory acceptable amount of (2*S*,4*R*)-4-methyl-2-((Z)-2-methylbut-1-en-1-yl)tetrahydro-2H-pyran the odor notes of a consumer product base will be improved, enhanced, or modified.

Thus, the invention furthermore provides a method for improving, enhancing or modifying a consumer product base by means of the addition thereto of an olfactorily acceptable amount of (2S,4R)-4-methyl-2-((Z)-2-methylbut-1-en-1-yl)tetrahydro-2H-pyran.

In one particular embodiment there is provided a method of replacing 4-methyl-2-(2-methylprop-1-en-1-yl)tetrahydro-2H-pyran in a fragrance composition, said method comprising employing in the fragrance composition (2S,4R)-4-methyl-2-((Z)-2-methylbut-1-en-1-yl)tetrahydro-2H-pyran, and at least one additional fragrance ingredient, so that the level of 4-methyl-2-(2-methylprop-1-en-1-yl)tetrahydro-2H-pyran, if present in said fragrance composition, is less than 0.1%, e.g. less than 0.01% or free of 4-methyl-2-(2-methylprop-1-en-1-yl)tetrahydro-2H-pyran.

The invention also provides a fragranced article comprising:

a) as odorant (2S,4R)-4-methyl-2-((Z)-2-methylbut-1-en-1-yl)tetrahydro-2H-pyran, and
b) a consumer product base.

As used herein, 'consumer product base' means a composition for use as a consumer product to fulfill specific actions, such as cleaning, softening, and caring or the like. Examples of such products include fine perfumery, e.g. perfume and eau de toilette; fabric care, household products and personal care products such as cosmetics, laundry care detergents, rinse conditioner, personal cleansing composition, detergent for dishwasher, surface cleaner; laundry products, e.g. softener, bleach, detergent; body-care products, e.g. shampoo, shower gel; air care products (includes products that contain preferably volatile and usually pleasant-smelling compounds which advantageously can even in very small amounts mask unpleasant odors). Air fresheners for living areas contain, in particular, natural and synthetic essential oils such as pine needle oils, citrus oil, eucalyptus oil, lavender oil, and the like, in amounts for example of up to 50% by weight. As aerosols they tend to contain smaller amounts of such essential oils, by way of example less than 5% or less than 2% by weight, but additionally include compounds such as acetaldehyde (in particular, <0.5% by weight), isopropyl alcohol (in particular, <5% by weight), mineral oil (in particular, <5% by weight), and propellants.

Cosmetic products include:

(a) cosmetic skincare products, especially bath products, skin washing and cleansing products, skincare products, eye makeup, lip care products, nail care products, intimate care products, foot care products;
(b) cosmetic products with specific effects, especially sunscreens, tanning products, de-pigmenting products, deodorants, antiperspirants, hair removers, and shaving products;
(c) cosmetic dental-care products, especially dental and oral care products, tooth care products, cleaners for dental

prostheses, adhesives for dental prostheses; and

(d) cosmetic hair care products, especially hair shampoos, hair care products, hair setting products, hair-shaping products, and hair coloring products.

This list of products is given by way of illustration, and is not to be regarded as being in any way limiting.

In one particular embodiment the consumer product base is selected form fine perfumery, and personal care products, including deodorants, hair care products, soaps, and the like.

In a further particular embodiment the consumer product base is selected from fabric care products, including fabric softener, and home care products, including air fresheners, dish washers and the like.

The invention is now further described with reference to the following non-limiting examples. These examples are for the purpose of illustration only and it is understood that variations and modifications can be made by one skilled in the art.

All products were purified after work-up by either flash chromatography (FC) using Tsingdao Haiyang Chemical silica gel (200 ± 300 mesh), silica gel Merck grade (60 Å) or distillation. Unless otherwise noted, a mixture of pentane : MTBE (10 : 1) was used as eluent. $^1$H and $^{13}$C NMR spectra were measured in $CDCl_3$ or $C_6D_6$. $^1$H NMR spetra in $CDCl_3$ were referenced to TMS (0.0 ppm) and the other ones to the residual hydrogen signal of the deuterated solvent ($^{13}$C 77.0 ppm in $CDCl_3$; $^1$H 7.16 ppm in $C_6D_6$, $^{13}$C 128.0 ppm in $C_6D_6$) and are reported as follows: chemical shifts (δ ppm), coupling constants J in Hz. GC-MS analyses were run on a MSD5975mass spectrometer and are reported as m/z list (relative intensity). Optical rotations were measured on a polarimeter MCP 200 from Anton Paar using a sample cell (100 mm long, 3 mm diameter) and a light source from Phillips 7388 6 V 20 W. Odor description refers to the odor of the isomeric mixture of the compounds unless otherwise indicated.

Definition of 4-methyl-2-(2-methylbut-1-en-1-yl)tetrahydro-2H-pyran isomers in this experimental section: 'cis-Z' means rel-(2S,4R)-(Z), 'cis-E' means rel-(2S,4R)-(E), 'trans-Z' means rel-(2R,4R)-(Z) and 'trans E' means rel-(2R,4R)-(E).

Example 1: Synthesis of (±)-4-methyl-2-(2-methylbut-1-en-1-yl)tetrahydro-2H-pyran

a) 4-Methyltetrahydro-2H-pyran-2-carbaldehyde

4-Methyl-2-(2-methylprop-1-en-1-yl)tetrahydro-2H-pyran (75.0 g, 486 mmol, 1.0 equiv, Rose oxide) was dissolved in MeOH (450 mL) and cooled to -70°C. Ozone was bubbled through the solution at -70 °C until all starting material was consumed according to GC (ca. 3 hours). After purging the solution with oxygen and argon, triethyl phosphite (107 ml, 598 mmol) was added dropwise (exoterm) at -20 to 0°C and the reaction mixture was stirred for 2 hours. The solvent was removed under reduced pressure to afford the crude aldehyde (180 g), which was purified twice by fractional distillation (81°C / 12 mbar) to give 4-methyltetrahydro-2H-pyran-2-carbaldehyde (34.3 g, 55% yield, mixture of diastereoisomers 97:3) as a slightly yelow oil.

$^1$H NMR (400 MHz, $CDCl_3$): δ 9.60 (d, J=0.7 Hz, 1H), 4.11 (ddd, J=11.5, 4.6, 1.6 Hz, 1H), 3.80 (dd, J=12.1, 2.6 Hz, 1H), 3.49 (ddd, J=12.4 ,11.5, 2.3 Hz, 1H), 1.92-1.83 (m, 1H), 1.76-1.55 (m, 2H), 1.35-1.20 (m, 1H), 1.09-0.95 (m, 1H), 1.00 (d, J=6.5 Hz, 3H) ppm. $^{13}$C NMR (101 MHz, $CDCl_3$): δ 201.3, 81.6, 67.9, 34.5, 34.0, 29.8, 22.1 ppm. GC-MS (EI, 70 eV) m/z (%): major isomer: 99 (99), 81 (25), 69 (17), 57 (12), 55 (39), 43 (100), 41 (50), 39 (29), 29 (65), 27 (31); minor isomer: 99 (100), 81 (25), 69 (16), 57 (10), 55 (30), 43 (70), 41 (35), 39 (18), 29 (47), 27 (20). bp 81°C (12 mbar).

Odor description: green, fatty, trans-2-hexenal, oily.

b) 4-Methyl-2-(2-methylbut-1-en-1-yl)tetrahydro-2H-pyran

To a suspension of sec-butyltriphenylphosphonium bromide (59.8 g, 150 mmol, 1.20 equiv) in THF (250 mL) was added dropwise at 0°C a solution of KOtBu (16.1 g, 144 mmol, 1.15 equiv) in THF (50 mL). After stirring for 1h at room temperature, a solution of 4-methyltetrahydro-2H-pyran-2-carbaldehyde (16.0 g, 125 mmol, 1.0 equiv) in THF (50 mL) was added dropwise at 0°C to the freshly prepared phosphonium ylide solution. After stirring for 2 h at 0°C, the reaction mixture was diluted with water (100 mL) and extracted with MTBE (250 mL). The combined organic extracts were washed with brine (70 mL), dried over MgSO4 and the solvent was evaporated. A solid precipitated out of the oil (triphenylphosphine oxide), which was filtred off after suspending the crude oil in hexanes. The filtrate was collected and the solvent was evaporated to give a crude product (20 g), which was Kugelrohr distilled (120°C / 8 mbar) to give 4-methyl-2-(2-methylbut-1-en-1-yl)tetrahydro-2H-pyran (16.0 g, 76% chemical yield). The product was repurified by fractional distillation (40°C / 0.1 mbar) to give 4-methyl-2-(2-methylbut-1-en-1-yl)tetrahydro-2H-pyran (14.0 g, 55% olfactive yield, mixture of four isomers cis-Z, cis-*E*, trans-Z and trans-E in the ratio of about 49 : 41 : 5 : 5, 90% cis, 10% trans) as a colorless oil.

Characteristic NMR signals for the two major isomers (cis-E and cis-Z) in the mixture: $^1$H NMR (400 MHz,

$CDCl_3$): δ 5.18-5.10 (m, 1H), 4.05 (m, 2H), 3.52-3.40 (m, 1H), 2.21-1.95 (m, 2H), 1.71 (d, J=1.4 Hz, 1.5H), 1.68 (d, J=1.3 Hz, 1.5H), 1.68-1.46 (m, 3H), 1.29-1.14 (m, 1H), 1.09-0.97 (m, 1H), 1.00 (2 × d, J=7.5 Hz and J=7.6 Hz, 3H), 0.94 (d, J=6.5 Hz, 1.5H), 0.93 (d, J=6.5 Hz, 1.5H) ppm. $^{13}C$ NMR (101 MHz, $CDCl_3$): δ 140.5 ($C_q$), 140.1 ($C_q$), 125.9 (CH), 124.6 (CH), 74.6 (CH), 74.1 (CH), 67.8 ($CH_2$), 67.8 ($CH_2$), 41.1 ($CH_2$), 40.8 ($CH_2$), 34.4 ($CH_2$), 34.3 ($CH_2$), 32.0 ($CH_2$), 30.2 (CH), 30.2 (CH), 25.4 ($CH_2$), 22.7 ($CH_3$), 22.2 (2x$CH_3$), 16.6 ($CH_3$), 13.0 ($CH_3$), 12.2 ($CH_3$) ppm. bp 40°C (0.1 mbar).

Odor description (isomeric mixture): metallic, mineral, rosy, rose oxide

Analytical data for pure cis-Z (rel-(2S,4R)-(Z)) after preparative GC:

$^{1}H$ NMR (600 MHz, $C_6D_6$): δ 5.42 (d, J=7.9 Hz, 1H), 4.05-3.99 (m, 1H), 3.96 (ddd, J=11.3, 4.5, 1.5 Hz, 1H), 3.29 (td, J=11.8, 2.3 Hz, 1H), 2.10-1.97 (m, 2H), 1.63 (d, J=1.1 Hz, 3H), 1.52-1.46 (m, 1H), 1.42-1.29 (m, 1H), 1.24-1.16 (m, 1H), 1.15-1.02 (m, 2H), 0.95 (t, J=7.5 Hz, 3H), 0.80 (d, J=6.4 Hz, 3H) ppm. $^{13}C$ NMR (151 MHz, $C_6D_6$): δ 138.9 ($C_q$), 127.7 (CH), 74.7 (CH), 67.7 ($CH_2$), 41.8 ($CH_2$), 34.9 ($CH_2$), 30.6 (CH), 25.9 ($CH_2$), 22.8 ($CH_3$), 22.6 ($CH_3$), 13.3 ($CH_3$) ppm. GC-MS (EI, 70 eV) m/z (%): 168 (2, $[M]^{+\cdot}$), 153 (8), 140 (10), 139 (100), 97 (9), 83 (19), 81 (9), 69 (43), 55 (20), 43 (9), 41 (20).

Odor description (cis-Z): rosy, rose oxide, metallic, strong

Analytical data for pure cis-E (rel-(2S,4R)-(E)) after preparative GC:

$^{1}H$ NMR (600 MHz, $C_6D_6$): δ 5.46 (d, J=7.9 Hz, 1H), 4.04-3.99 (m, 1H), 3.97 (ddd, J=11.7, 4.6, 1.1 Hz, 1H), 3.30 (td, J=11.9, 2.3, 1H), 1.92 (q, J=7.5 Hz, 2H), 1.60 (d, J=0.8 Hz, 3H), 1.52-1.46 (m, 1H), 1.41-1.30 (m, 1H), 1.24-1.18 (m, 1H), 1.13 (td, J=12.4, 4.5 Hz, 1H), 1.09-1.1 (m, 1H), 0.95 (t, J=7.5 Hz, 3H), 0.81 (d, J=6.4 Hz, 3H) ppm. $^{13}C$ NMR (151 MHz, $C_6D_6$): δ 138.4 ($C_q$), 126.3 (CH), 75.1 (CH), 67.8 ($CH_2$), 41.3 ($CH_2$), 34.8 ($CH_2$), 32.3 ($CH_2$), 30.6 (CH), 22.4 ($CH_3$), 16.6 ($CH_3$), 12.6 ($CH_3$) ppm. GC-MS (EI, 70 eV) m/z (%): 168 (2, $[M]^{+\bullet}$), 153 (7), 140 (10), 139 (100), 97 (9), 83 (18), 81 (8), 69 (42), 55 (20), 43 (9), 41 (19).

Odor description (cis-*E*): rosy, rose oxide, metallic, weak

Analytical data for pure trans-Z (rel-(2R,4R)-(Z)) after preparative GC:

GC-MS (EI, 70 eV) m/z (%): 168 (1, $[M]^{+\bullet}$), 140 (10), 139 (100), 97 (9), 83 (20), 81 (11), 69 (44), 55 (22), 43 (10), 41 (20), 39 (8).

Odor description (trans-Z*)*: very weak, almost odorless

Analytical data for pure trans-E (rel-(2R,4R)-(E)) after preparative GC:

$^{1}H$ NMR (600 MHz, $C_6D_6$): δ 5.50 (d, J=7.2 Hz, 1H), 4.54-4.48 (m, 1H), 3.74-3.67 (m, 1H), 3.65-3.58 (m, 1H), 1.93 (q, J=7.6 Hz, 2H), 1.84-1.74 (m, 1H), 1.63 (s, 3H), 1.61-1.53 (m, 2H), 1.39-1.32 (m, 1H), 1.10-1.03 (m, 1H), 0.95 (t, J=7.5 Hz, 3H), 0.89 (d, J=7.2 Hz, 3H). $^{13}C$ NMR (151 MHz, $C_6D_6$): δ 139.9 (Cq), 124.8 (CH), 69.3 (CH), 61.9 ($CH_2$), 38.6 ($CH_2$), 33.3 ($CH_2$), 32.5 ($CH_2$), 25.4 (CH), 19.8 ($CH_3$), 16.5 ($CH_3$), 12.7 ($CH_3$) ppm. GC-MS (EI, 70 eV) m/z (%): 168 (1, $[M]^{+\bullet}$), 140 (10), 139 (100), 97 (9), 83 (18), 81 (9), 69 (44), 55 (21), 43 (10), 41 (18), 39 (7).

Odor description (trans-E): slightly floral, rose oxide, very weak

Example 2: Synthesis of (4R)-4-methyl-2-(2-methylbut-1-en-1-yl)tetrahydro-2H-pyran

a) (4R)-4-Methyltetrahydro-2H-pyran-2-carbaldehyde

Following the procedure described in Example 1a, (4R)-4-methyl-2-(2-methylprop-1-en-1-yl)tetrahydro-2H-pyran (10.0 g, 64.8 mmol, Rose oxide Laevo, cis/trans 69/31, 57 % ee, $[\alpha]_D^{25}$ = -25.9° (c = 2.635, EtOH)) was ozonized to give (4R)-4-methyltetrahydro-2H-pyran-2-carbaldehyde (4.5 g, 54%, mixture of diastereoisomers 81:19) as a colorless oil.

$$[\alpha]_D^{32} = -11.0° \text{ (c = 0.955, EtOH).}$$

Odor description: green, fatty, oily.

b) (4R)-4-methyl-2-(2-methylbut-1-en-1-yl)tetrahydro-2H-pyran

Following the procedure described in Example 1b, (4R)-4-methyltetrahydro-2H-pyran-2-carbaldehyde (2.2 g, 17.2 mmol) was subjected to a Wittig reaction to give (4R)-4-methyl-2-(2-methylbut-1-en-1-yl)tetrahydro-2H-pyran (1.7 g, 59% olfactive yield, mixture of four isomers cis-Z, cis-E, trans-Z, and trans-E in a ratio of 50:36:8:6; 86% cis, 14% trans, 58% ee) as a colorless oil.

$$[\alpha]_D^{30} = -28.3° \text{ (c = 1.235, EtOH).}$$

(2R,4S)-4-methyl-2-((Z)-2-methylbut-1-en-1-yl)tetrahydro-2 H-pyran: Chiral GC (Astec CHIRALDEX G-DP), 50 °C → 2 °C/min → 200 °C $H_2$): $t_R$/min = 19.7.
Odor description: very weak, almost odorless
(2S,4R)-4-methyl-2-((Z)-2-methylbut-1-en-1-yl)tetrahydro-2H-pyran: Chiral GC (Astec CHIRALDEX G-DP), 50 °C → 2 °C/min → 200 °C $H_2$): $t_R$/min = 20.3.
Odor description: rosy, rose oxide, metallic, strong
(2R,4S)-4-methyl-2-((E)-2-methylbut-1-en-1-yl)tetrahydro-2 H-pyran: Chiral GC (Astec CHIRALDEX G-DP), 50 °C → 2 °C/min → 200 °C $H_2$): $t_R$/min = 21.2.
Odor description: very weak, almost odorless
(2S,4R)-4-methyl-2-((E)-2-methylbut-1-en-1-yl)tetrahydro-2H-pyran: Chiral GC (Astec CHIRALDEX G-DP), 50 °C → 2 °C/min → 200 °C $H_2$): $t_R$/min = 21.9.
Odor description: rosy, rose oxide, metallic, weak
(2R,4R)-4-methyl-2-((Z)-2-methylbut-1-en-1-yl)tetrahydro-2H-pyran: Chiral GC (Astec CHIRALDEX G-DP), 50 °C → 2 °C/min → 200 °C $H_2$): $t_R$/min = 20.8.
Odor description: very weak, almost odorless
(2S,4S)-4-methyl-2-((Z)-2-methylbut-1-en-1-yl)tetrahydro-2H-pyran: Chiral GC (Astec CHIRALDEX G-DP), 50 °C → 2 °C/min → 200 °C $H_2$): $t_R$/min = 21.1.
Odor description: very weak, almost odorless
(2R,4R)-4-methyl-2-((E)-2-methylbut-1-en-1-yl)tetrahydro-2H-pyran: Chiral GC (Astec CHIRALDEX G-DP), 50 °C → 2 °C/min → 200 °C $H_2$): $t_R$/min = 21.7.
Odor description: slightly floral, rose oxide, very weak.
(2S,4S)-4-methyl-2-((E)-2-methylbut-1-en-1-yl)tetrahydro-2H-pyran: Chiral GC (Astec CHIRALDEX G-DP), 50 °C → 2 °C/min → 200 °C $H_2$): $t_R$/min = 22.2.
Odor description: very weak, almost odorless

<u>Example 3: Toxicity study</u>

An isomeric mixture obtained according to Example 1b was tested in a repeated dose 28-day oral toxicity study in rats according to OECD Guideline 407 (OECD, Paris, October 2008). Groups of animals (n=5) were fed with a diet containing the test item to obtain a daily ingested dose of 100 mg/kg body weight / day, 300 mg/kg body weight / day and 1000 mg/kg body weight / day. The animals were treated for 28 days. After this period, they were sacrificed and their organs were examined by pathological examination for gross lesions, weight changes and further investigated by histopathology. The seminal fluid from the epididymis was sampled and analyzed microscopically for number, morphology and motility of sperms.
The weight of the reproductive organs was not different from the control group and no macroscopic changes to reproductive organs were noted. The results of the sperm analysis can be found in Table 1 below.

Table 1. Analysis of sperm parameters in male rats (n=5 per group) treated with an isomeric mixture obtained according to Example 1b for 28 days.

| Endpoint assessed | Group 1: Control | Group 2: 100 mg/kg bw/d | Group 2: 300 mg/kg bw/d | Group 2: 1000 mg/kg bw/d |
|---|---|---|---|---|
| Number of sperms per gram | $3.0 \pm 0.9 \times 10^8$ | $3.9 \pm 0.9 \times 10^8$ | $4.0 \pm 0.9 \times 10^8$ | $4.0 \pm 1.9 \times 10^8$ |
| Sperm cells with normal morphology | 174/225 (77.3%) | 156/246 (63.4%) | 170/210 (81.0%) | 158/197 (80.2%) |
| Motile Sperms (%) | 72 ± 15 % | 63 ± 12 % | 47 ± 13 % | 53 ± 23 % |
| bw: body weight (mg/kg bw/d = mg/kg body weight per day) | | | | |

All treated groups were compared to the control group with a Dunnett-test based on pooled variance and a Steel-test. No significant differences between control group and treated groups were found at a $p$=0.05 significance level.
Based on these results, it has been shown that an isomeric mixture obtained according to Example 1b has no effect on key parameters of male fertility when tested for 28 days and up to a high dose of 1000 mg/ kg bw / d. This can be compared to the effects of an isomeric mixture of Rose oxide, which had been tested for 28 days with an equivalent test (OECD guideline 407), showing clear-cut effects on sperm formation both at the middle (300 mg/ kg bw/d) and high (1000 mg/kg bw/d) dose.

Example 3: rosy fragrance accord

| Compound / Ingredient | parts by weight 1/1000 |
|---|---|
| BENZYL METHYL ETHER | 0.3 |
| CEPIONATE (methyl 3-oxo-2-pentylcyclopentaneacetate) | 10 |
| CITRAL LEMAROME N (3,7-DIMETHYL-2,6-OCTADIENAL) | 6 |
| CITRONELLOL (3,7-DIMETHYL-6-OCTEN-1-OL) | 300 |
| CITRONELLYL ACETATE (3,7-DIMETHYL-6-OCTEN-1-YL ACETATE) | 115 |
| DAMASCENONE (1-(2,6,6- TRIMETHYL-1,3-CYCLOHEXADIENE-1-YL)-2-BUTENE-1-ONE) | 1 |
| DAMASCONE ALPHA (1-(2,6,6-TRIMETHYL-2-CYCLOHEXEN-1-YL)-2-BUTEN-1-ONE) | 1 |
| DIHYDRO FARNESAL (3,7,11-TRIMETHYL-6,10-DODECADIENAL) | 5 |
| DIHYDRO IONONE BETA (4-(2,6,6- T RIM ETHYL- 1-CYCLOHEXEN-1-YL) BUTAN-2-O N E) | 7 |
| GERANIOL (2-TRANS-3,7-DIMETHYL-2,6-OCTADIEN-1-OL) | 180 |
| GERANYL ACETATE (3,7-DIMETHYLOCTA-2,6-DIENYL ACETATE) | 80 |
| GRAPEFRUIT OIL | 1 |
| HEXENAL-2-TRANS 10%/TEC | 0.3 |
| CIS-3-HEXENYL ACETATE | 3 |
| HEXYL ACETATE | 2 |
| IONONE BETA (4-(2,6,6-TRIMETHYL-1-CYCLOHEXEN-1-YL)-3-BUTEN-2-ONE) | 3 |
| ISO E SUPER® | 50 |
| ISOAMYL ACETATE (3-methylbut-1-yl acetate) | 0.8 |
| LABIENOXIME 1%/IPM-TEC1) | 3 |
| LINALOOL (3,7-DIMETHYL-1,6-OCTADIEN-3-OL) | 5 |
| NEROLEX (3,7-DIMETHYL-2,6-OCTADIEN-1-OL) | 30 |
| PHENYL ETHYL ACETATE (2-PHENYLETHYL ACETATE) | 5 |
| PHENYL ETHYL ALCOHOL (2-PHENYLETHANOL) | 10 |
| PRENYL ACETATE (3-METHYL-2-BUTEN-1-YL ACETATE) | 0.8 |
| THIBETOLIDE (CYCLOPENTADECANOLIDE) | 50 |
| ROSE OXIDE | 3 |
| DIPROPYLENE GLYCOL (DPG) | 127.8 |
| | Total 1000 |

1) 2,4,4,7-TETRAMETHYL-6,8-NONADIENE-3-ONE OXIME @1% in isopropylmyristate/triethylcitrate (50/50mixture)

The accord above represents a rosy, natural, fresh, fruity accord with green leafy and green metallic geranium

rosy aspects, suitable for fine perfumery at a dosage of e.g. 5 weight %.

The replacement of Rose Oxide by (2*S*,4*R*)-4-methyl-2-((Z)-2-methylbut-1-en-1-yl)tetrahydro-2H-pyran (isomeric mixture obtained according to Example 1b) was not noticeable by the perfumers. Accordingly, (2*S*,4*R*)-4-methyl-2-((Z)-2-methylbut-1-en-1-yl)tetrahydro-2H-pyran mimics quite closely both, the olfactive contribution and the evaporation profile of Rose Oxide.

## Claims

1. (2*S*,4*R*)-4-Methyl-2-((Z)-2-methylbut-1-en-1-yl)tetrahydro-2H-pyran of formula 1'a

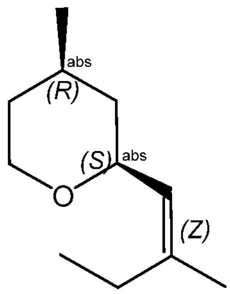

(1'a)

2. A fragrance composition comprising

   a. (2*S*,4*R*)-4-methyl-2-((Z)-2-methylbut-1-en-1-yl)tetrahydro-2H-pyran, and
   b. at least one additional fragrance ingredient.

3. A fragrance composition according to claim 2 **characterized in that** the fragrance composition contains less than 0.1% by weight of 4-methyl-2-(2-methylprop-1-en-1-yl)tetrahydro-2H-pyran.

4. A fragrance composition according to any one of the claims 2 to 3 **characterized in that** the fragrance composition is free of 4-methyl-2-(2-methylprop-1-en-1-yl)tetrahydro-2H-pyran.

5. A fragrance composition comprising (2*S*,4*R*)-4-methyl-2-((Z)-2-methylbut-1-en-1-yl)tetrahydro-2H-pyran and at least one additional fragrance ingredient, wherein said composition is free of 4-methyl-2-(2-methylprop-1-en-1-yl)tetrahydro-2H-pyran, wherein the composition is a replacement for 4-methyl-2-(2-methylprop-1-en-1-yl)tetrahydro-2H-pyran with respect to having a similar odor profile, while reducing the toxic effects, in particular reducing the risk of sperm formation suppression to the consumer.

6. A fragranced article t comprising (2*S*,4*R*)-4-methyl-2-((Z)-2-methylbut-1-en-1-yl)tetrahydro-2H-pyran or a fragrance composition as defined in one of the claims 2 to 5, and a consumer product base.

7. A fragranced article according to claim 6 wherein the consumer product base is selected from perfumery, fabric care, household products and personal care products.

8. A fragranced article according to claim 7 wherein the personal care product is selected from deodorant products, hair care products and soaps.

9. Use of (2*S*,4*R*)-4-methyl-2-((Z)-2-methylbut-1-en-1-yl)tetrahydro-2H-pyran as a replacement for 4-methyl-2-(2-methylprop-1-en-1-yl)tetrahydro-2H-pyran.

10. A method of replacing 4-methyl-2-(2-methylprop-1-en-1-yl)tetrahydro-2H-pyran in a fragrance composition, said method comprising employing in the fragrance composition (2*S*,4*R*)-4-methyl-2-((Z)-2-methylbut-1-en-1-yl)tetrahydro-2H-pyran, and at least one additional fragrance ingredient, so that the level of 4-methyl-2-(2-methylprop-1-en-1-yl)tetrahydro-2H-pyran, if present in said fragrance composition is less than 0.1% by weight.

11. A method according to claim 10 wherein said fragrance composition is free of 4-methyl-2-(2-methylprop-1-en-1-yl)tetrahydro-2H-pyran.

## Patentansprüche

1. (2*S*,4*R*)-4-Methyl-2-((Z)-2-methylbut-1-en-1-yl)-tetrahydro-2H-pyran der Formel I'a

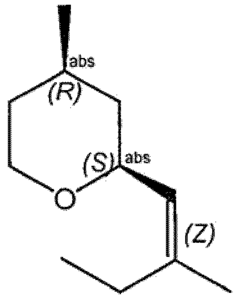

(1'a)

2. Duftstoffzusammensetzung, umfassend

   a. (2*S*,4*R*)-4-Methyl-2-((Z)-2-methylbut-1-en-1-yl)-tetrahydro-2H-pyran und
   b. mindestens einen zusätzlichen Duftstoffbestandteil.

3. Duftstoffzusammensetzung nach Anspruch 2, **dadurch gekennzeichnet, dass** die Duftstoffzusammensetzung weniger als 0,1 Gew.-% 4-Methyl-2-(2-methylprop-1-en-1-yl)tetrahydro-2H-pyran enthält.

4. Duftstoffzusammensetzung nach einem der Ansprüche 2 bis 3, **dadurch gekennzeichnet, dass** die Duftstoffzusammensetzung frei von 4-Methyl-2-(2-methylprop-1-en-1-yl)tetrahydro-2H-pyran ist.

5. Duftstoffzusammensetzung, umfassend (2*S*,4*R*)-4-Methyl-2-((Z)-2-methylbut-1-en-1-yl)tetrahydro-2H-pyran und mindestens einen zusätzlichen Duftstoffbestandteil, wobei die Zusammensetzung frei von 4-Methyl-2-(2-methylprop-1-en-1-yl)tetrahydro-2H-pyran ist, wobei die Zusammensetzung ein Ersatz für 4-Methyl-2-(2-methylprop-1-en-1-yl)tetrahydro-2H-pyran in Bezug auf das Aufweisen eines ähnlichen Geruchsprofils ist und dabei die toxischen Effekte verringert, insbesondere das Risiko der Unterdrückung der Spermienbildung für den Verbraucher, verringert.

6. Duftstoffhaltiger Artikel, umfassend (2S,4R)-4-Methyl-2-((Z)-2-methylbut-1-en-1-yl)tetrahydro-2H-pyran oder eine Duftstoffzusammensetzung gemäß einem der Ansprüche 2 bis 5 und eine Konsumproduktbasis.

7. Duftstoffhaltiger Artikel nach Anspruch 6, wobei die Konsumproduktbasis aus Parfümerie, Textilpflege-, Haushaltsprodukten und Körperpflegeprodukten ausgewählt ist.

8. Duftstoffhaltiger Artikel nach Anspruch 7, wobei das Körperpflegeprodukt aus Deodorantprodukten, Haarpflegeprodukten und Seifen ausgewählt ist.

9. Verwendung von (2*S*,4*R*)-4-Methyl-2-((Z)-2-methylbut-1-en-1-yl)tetrahydro-2H-pyran als Ersatz für 4-Methyl-2-(2-methylprop-1-en-1-yl)tetrahydro-2H-pyran.

10. Verfahren zum Ersetzen von 4-Methyl-2-(2-methyl-prop-1-en-1-yl)tetrahydro-2H-pyran in einer Duftstoffzusammensetzung, bei dem man in der Duftstoffzusammensetzung (2*S*,4*R*)-4-Methyl-2-((Z)-2-methyl-but-1-en-1-yl)tetrahydro-2H-pyran und mindestens einen zusätzlichen Duftstoffbestandteil derart einsetzt, dass der Gehalt an 4-Methyl-2-(2-methyl-prop-1-en-1-yl)tetrahydro-2H-pyran, sofern es in der Duftstoffzusammensetzung vorliegt, weniger als 0,1 Gew.-% beträgt.

11. Verfahren nach Anspruch 10, wobei die Duftstoffzusammensetzung frei von 4-Methyl-2-(2-methylprop-1-en-1-yl)tetrahydro-2H-pyran ist.

## Revendications

1. (2*S*,4*R*)-4-Méthyl-2-((Z)-2-méthylbut-1-én-1-yl)tétrahydro-2H-pyranne de formule I'a

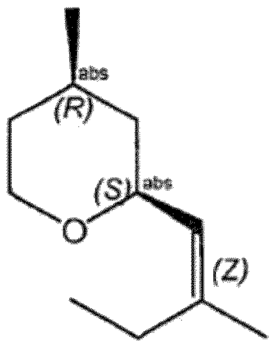

(1'a)

2. Composition de fragrance comprenant

a. du (2*S*,4*R*)-4-méthyl-2-((Z)-2-méthylbut-1-én-1-yl)tétrahydro-2H-pyranne, et
b. au moins un ingrédient de fragrance supplémentaire.

3. Composition de fragrance selon la revendication 2 **caractérisée en ce que** la composition de fragrance contient moins de 0,1 % en poids de 4-méthyl-2-(2-méthylprop-1-én-1-yl)tétrahydro-2H-pyranne.

4. Composition de fragrance selon l'une quelconque des revendications 2 et 3 **caractérisée en ce que** la composition de fragrance est exempte de 4-méthyl-2-(2-méthylprop-1-én-1-yl)tétrahydro-2H-pyranne.

5. Composition de fragrance comprenant du (2*S*,4*R*)-4-méthyl-2-((Z)-2-méthylbut-1-én-1-yl)tétrahydro-2H-pyranne et au moins un ingrédient de fragrance supplémentaire, ladite composition étant exempte de 4-méthyl-2-(2-méthylprop-1-én-1-yl)tétrahydro-2H-pyranne, la composition étant un remplacement du 4-méthyl-2-(2-méthylprop-1-én-1-yl)tétrahydro-2H-pyranne par rapport au fait qu'elle possède un profil olfactif similaire, tout en réduisant les effets toxiques, en particulier en réduisant le risque de suppression de formation de sperme au consommateur.

6. Article fragrancé comprenant du (2*S*,4*R*)-4-méthyl-2-((Z)-2-méthylbut-1-én-1-yl)tétrahydro-2H-pyranne ou une composition de fragrance telle que définie dans l'une des revendications 2 à 5, et une base de produit de consommation.

7. Article fragrancé selon la revendication 6, la base de produit de consommation étant choisie parmi des produits de parfumerie, d'entretien de tissus, ménagers et des produits de soin personnel.

8. Article fragrancé selon la revendication 7, le produit de soin personnel étant choisi parmi des produits déodorants, des produits de soin capillaire et des savons.

9. Utilisation du (2*S*,4*R*)-4-méthyl-2-((Z)-2-méthylbut-1-én-1-yl)tétrahydro-2H-pyranne en tant que remplacement du 4-méthyl-2-(2-méthylprop-1-én-1-yl)tétrahydro-2H-pyranne.

10. Procédé de remplacement du 4-méthyl-2-(2-méthylprop-1-én-1-yl)tétrahydro-2H-pyranne dans une composition de fragrance, ledit procédé comprenant l'emploi dans la composition de fragrance de (2*S*,4*R*)-4-méthyl-2-((Z)-2-méthylbut-1-én-1-yl)tétrahydro-2H-pyranne, et d'au moins un ingrédient de fragrance supplémentaire, de sorte que le taux de 4-méthyl-2-(2-méthylprop-1-én-1-yl)tétrahydro-2H-pyranne, s'il est présent dans ladite composition de fragrance, soit inférieure à 0,1 % en poids.

11. Procédé selon la revendication 10, ladite composition de fragrance étant exempte de 4-méthyl-2-(2-méthylprop-1-én-1-yl)tétrahydro-2H-pyranne.